# EUROPEAN PATENT APPLICATION

(11) **EP 4 312 227 A1**
(43) Date of publication of application: **31.01.2024**
(21) Application number: 22186780.7
(22) Date of filing: 25.07.2022
(51) Int. Cl.: G16H 40/63, A61B 5/00, G16H 50/30

(54) **TRAIL MAKING TEST SYSTEM**

(71) Applicant: Koninklijke Philips N.V., 5656 AG Eindhoven (NL)
(72) Inventor: VAN DOOREN, Marieke, Eindhoven (NL); VAN EE, Raymond, Eindhoven (NL); VOGT, Juergen, 5656AG Eindhoven (NL); SPELT, Hanne Adriana Alijda, 5656AG Eindhoven (NL)
(74) Representative: Philips Intellectual Property & Standards

(57) **Abstract**

The present invention relates to a Trail Making Test system (10), comprising:
- an input/output unit (20); and
- a processing unit (30);
wherein the input/output unit is configured to present a calibration Trail Making Test to a user, wherein presentation of the calibration Trail Making Test comprises a plurality of targets being presented on the input/output unit, wherein to undertake the calibration Trail Making Test the user is requested to trace a trail linking the plurality of targets in a specific order, and wherein a task difficulty level increases progressively with respect to the trail linking the plurality of targets in the specific order;
wherein the input/output unit is configured to detect input from the user as they trace a trail between targets as they undertake at least some of the calibration Trail Making Test;
wherein the processing unit is configured to determine or select an evaluation Trail Making Test for the user comprising utilization of the detected input from the user; and
wherein the input/output unit is configured to present the evaluation Trail Making Test on the input/output unit, wherein presentation of the evaluation Trail Making Test comprises a plurality of targets being presented on the input/output unit, wherein to undertake the evaluation Trail Making Test the user is requested to trace a trail linking the plurality of targets in a specific order.

## Description

### FIELD OF THE INVENTION

The present invention relates to Trail Making Test (TMT) systems, Trail Making Test (TMT) methods, as well as to a computer program element and a computer readable medium.

### BACKGROUND OF THE INVENTION

Information regarding the Trail Making Test can be found in the following article: Vermeent, S., de Oliveira, M. & Schmand, B. (2020). "Which cognitive processes are involved in the Trail Making Test? Towards a more precise neuropsychological assessment using digital tablet-based technology".

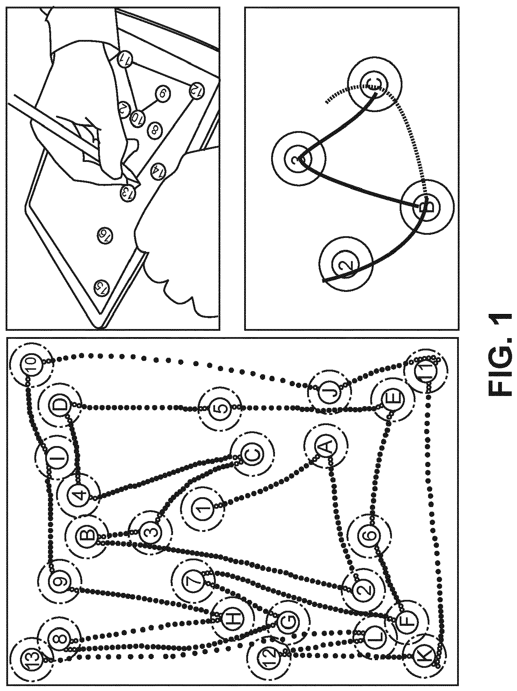

Philips Healthcare has automatized the paper and pencil version of the Trail Making Test and made it accessible in digital tablet-based technology. Therefore, it becomes technically possible to track the actual behaviour of a participant continuously in space (x, y coordinates) and in time (time to reach a target, time to dwell on a target, total path time...). These features allow for more precise performance measurement. Fig. 1 shows The Trail Making Test in digital tablet-based technology as implemented by Digital Cognitive Diagnostics, Philips Healthcare.

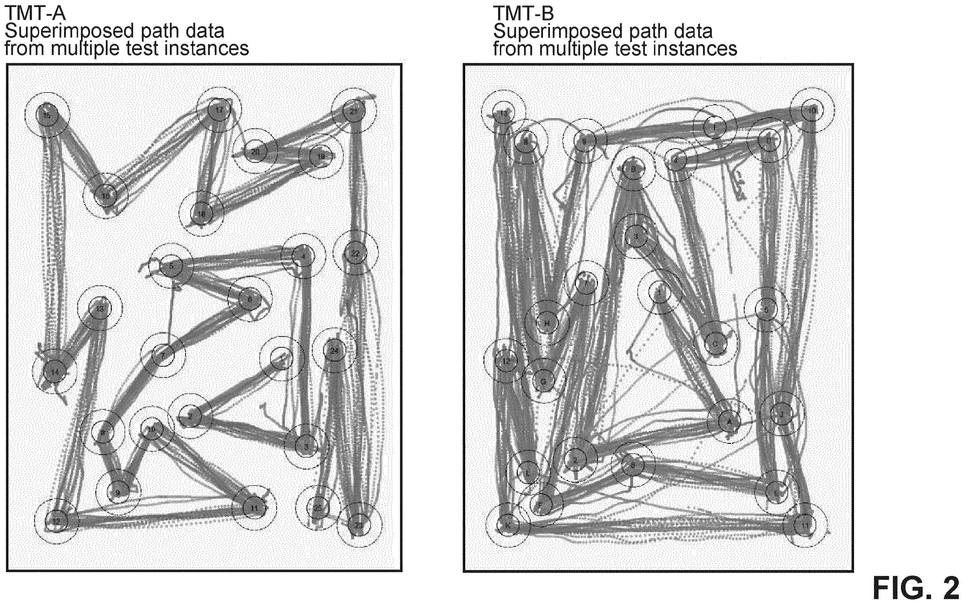

The standard version of the Trail Making Test has a TMT-A and a TMT-B as illustrated in Fig. 2, which shows an illustration of the TMT-A on the left side, and the TMT-B on the right side.

TMT-A consists of only numbers that should be connected. from 1 → 2 → 3 → 4, ... TMT-B consists of numbers and letters that have to be connected in order, from 1 → A → 2 → B → 3 → C, ...

It has investigated what makes TMT-B more difficult, (see: Elizabeth A. Gaudino , Mark W. Geisler & Nancy K. Squires (1995) Construct validity in the trail making test: What makes part B harder?, Journal of Clinical and Experimental Neuropsychology, 17:4, 529-535) and this has been linked to:
- increased trail length (as distance between targets in cm) and
- increased amount of visual interference (as counting the number of targets not in sequence that lie within 3 cm of the path between each target)

Therefore, TMT-B is much more difficult and requires more executive functions.

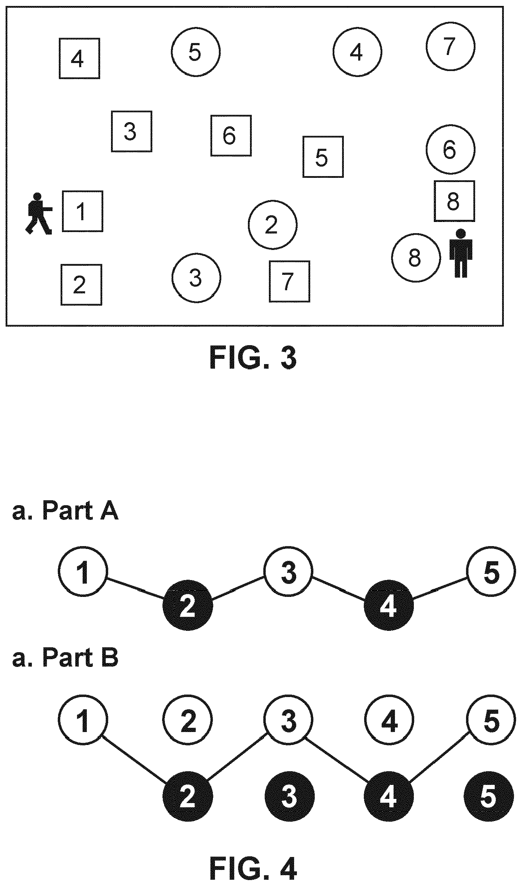

Several different variants of the original TMT-A and TMT-B have been proposed. For example, in certain cultures a different alphabet is used and the connections with letters in the TMT-B is not easy because this is traditionally done with A-B-C-D-... Fig. 3 shows an example of aShape Trail Test with circles and rectangles (from: Zhao, Q. et al. (2013), Plos One, Vol. 8 (2). The Shape Trail Test: Application of a New Variant of the Trail Making Test). Fig. 4 shows an example of a Colours Trail Making Test in black & white (from: Kim, H.J. (2014), Plos One, Vol. 9 (2). Alternative Type of the Trail Making Test in Nonnative English-Speakers: The Trail Making Test-Black & White). Thus, in some TMTs, the letters have been replaced by circular and rectangular numbers, and in some TMTs a Colour Trail Making Test is being used (and validated), where for example black and white numbers are connected subsequently.

For subjects with neuropsychological problems it often takes a prolonged period of time to administer the TMT task, and some subjects or users do not succeed in accomplishing the task in a limited time window (See: Harciarek, M. et al. (2017). Assessment of Executive Function in Dementia, in Executive Functions in Health and Disease, 2017, Chapter 19. Dementias and the Frontal Lobes). An incomplete task provides only limited information and makes it almost impossible to track and quantify progress (or decline) longitudinally (over time).

Also, although various alternatives have been proposed of the original paper-and-pencil version of the test, a test that is personalized for a particular participant has not been developed.

There is a need to address these issues.

Further details on the TMT can be found for example in:
L. Klaming and B.N.S. Vlaskamp, Non-dominant hand use increases completion time on part B of the Trail Making Test but not on part A, Behaviour Research Methods 50, 1074-1087 (2018).
A. Poreh et al, Decomposition of the Trail Making Test - Reliability and Validity of a Computer Assisted Method for Data Collection, Archives of assessment Psychology vol 2 No 1 (2012).
L.M. McCracken and M.D. Franzen, Principal-components analysis of the equivalence of alternate forms of the Trail Making Test, Psychological Assessment, vol 4(2) 235-238 (1992).
S. Wagner et al, Reliability of Three Alternate Forms of the Trail Making Tests A and B, Archives of Clinical Neuropsychology, vol 26 Issue 4, 314-321 (2011).

### SUMMARY OF THE INVENTION

It would be advantageous to provide an improved Trail Making Test that is personalized for the participate, or patient, hereafter generally referred to as user.

The object of the present invention is solved with the subject matter of the independent claims, wherein further embodiments are incorporated in the dependent claims. It should be noted that the following described aspects and examples of the invention apply to the Trail Making Test systems the Trail Making Test methods, as well as to a computer program element and a computer readable medium, and have applicability to all types of TMTs, for example as outline in the background section above.

In a first aspect, there is provided a Trail Making Test system, comprising:
- an input/output unit; and
- a processing unit.

The input/output unit is configured to present a calibration Trail Making Test to a user. The presentation of the calibration Trail Making Test comprises a plurality of targets being presented on the input/output unit. To undertake the calibration Trail Making Test the user is requested to trace a trail linking the plurality of targets in a specific order, and a task difficulty level increases progressively with respect to the trail linking the plurality of targets in the specific order. The input/output unit is configured to detect input from the user as they trace a trail between targets as they undertake at least some of the calibration Trail Making Test. The processing unit is configured to determine or select an evaluation Trail Making Test for the user comprising utilization of the detected input from the user. The input/output unit is configured to present the evaluation Trail Making Test on the input/output unit. The presentation of the evaluation Trail Making Test comprises a plurality of targets being presented on the input/output unit. To undertake the evaluation Trail Making Test the user is requested to trace a trail linking the plurality of targets in a specific order.

In an example, the processing unit is configured to utilize the detected input from the user to determine a task difficulty level for the user. The processing unit is configured to determine or select the evaluation Trail Making Test for the user comprising utilization of the determined task difficulty level for the user.

In an example, the processing unit is configured to determine the task difficulty level for the user as the task difficulty level associated with adjacent targets of the trail linking the plurality of targets in the specific order, and the processing unit is configured to determine the adjacent targets for the determination of the task difficulty level. The determination of the adjacent targets for the determination of the task difficulty level comprises a determination that the user exceeds a threshold time to trace a trail on the input/output unit linking the adjacent targets or a determination that the user fails to trace a trail between the adjacent targets within the threshold time.

In an example, the input/output unit is configured to detect brain activity of the user as they trace the trail between targets as they undertake at least some of the calibration Trail Making Test. The processing unit is configured to determine the task difficulty level for the user as the task difficulty level associated with adjacent targets of the trail linking the plurality of targets in the specific order, and the processing unit is configured to determine the adjacent targets comprising utilization of the brain activity of the user as they undertake at least some of the calibration Trail Making Test.

In an example, the processing unit is configured to select the evaluation Trail Making Test from a plurality of Trail Making Tests each having a different task difficulty level as a Trail Making Test of the plurality of Trail Making Tests that has a difficulty level that most closely matches the determined task difficulty level for the user.

In an example, the processing unit is configured to determine the evaluation Trail Making Test by adaptation of a reference Trail Making Test to have a difficulty level that corresponds to the determined task difficulty level for the user.

In a second aspect, there is provided a Trail Making Test system, comprising:
- an input/output unit; and
- a processing unit.

The input/output unit is configured to present a subset of a Trail Making Test to a user. The presentation of the subset of the Trail Making Test comprises a subset of a plurality of targets being presented on the input/output unit. To undertake the subset of the Trail Making Test the user is requested to trace a trail linking the subset of the plurality of targets in a specific order. The input/output unit is configured to detect input from the user as they trace a trail between targets as they undertake the subset of the Trail Making Test. The processing unit is configured to determine a performance level for the user, the determination comprising utilization of the detected input from the user. The processing unit is configured to:
if the determined performance level matches a required performance level present the remainder of the Trail Making Test to the user. The presentation of the remainder of the Trail Making Test comprises a remainder of the plurality of targets being presented on the input/output unit. To undertake the remainder of the Trail Making Test the user is requested to trace a trail linking the remainder of the plurality of targets in a specific order;
if the determined performance level does not match the required performance level carry out the following until the determined performance level matches the required performance level: adapt the Trail Making Test, wherein the adaptation comprises utilization of the determined performance level;
for the adapted Trail Making Test present of a subset of the Trail Making Test to the user, wherein presentation of the subset of the Trail Making Test comprises a subset of a plurality of targets being presented on the input/output unit, wherein to undertake the subset of the Trail Making Test the user is requested to trace a trail linking the subset of the plurality of targets in a specific order;
for the adapted Trail Making Test detect input from the user as they trace a trail between targets as they undertake the subset of the Trail Making Test;
for the adapted Trail Making Test determine a performance level for the user, the determination comprising utilization of the detected input from the user; and
for the adapted Trail Making Test when the determined performance level matches the required performance level present the remainder of the adapted Trail Making Test to the user.

In an example, the processing unit is configured to utilize the detected input from the user as they trace the trail between targets as they undertake the subset of the Trail Making Test to determine a time for the user to complete the subset of the Trail Making Test. The determined performance level comprises the time for the user to complete the subset of the Trail Making Test. The processing unit is configured to determine that the determined performance level matches the required performance level comprising a determination that the time for the user to complete the subset of the Trail Making Test is within a threshold time of an ideal time for the user to complete a subset of a Trail Making Test.

In an example, the processing unit is configured to adapt the Trail Making Test to increase a task difficulty level of the Trail Making Test if the time for the user to complete the subset of the Trail Making Test is below the ideal time for the user to complete a subset of a Trail Making Test by more than the threshold time. The processing unit is configured to adapt the Trail Making Test to decrease a task difficulty level of the Trail Making Test if the time for the user to complete the subset of the Trail Making Test is above the ideal time for the user to complete a subset of a Trail Making Test by more than the threshold time.

In an example, adaptation of the Trail Making Test comprises one or more of: a change in distance between two or more of the targets of the plurality of targets in the specific order when presented on the input/output unit; a change in a number of targets not in sequence that lie within a threshold distance of the trail linking the plurality of targets when presented on the input/output unit; a change in the symmetry of the plurality of targets when presented on the input/output unit; a change in intensity level and/or colour between adjacent targets when presented on the input/output unit.

In an example, the processing unit is configured to determine from the input from the user as they trace a trail between targets as they undertake the subset of the Trail Making Test that the user has a deficiency with respect to a specific presentation area of the input/output unit less than the overall presentation area of the input/output unit. In this case, for the adapted Trail Making Test, none of the subset of the plurality of targets or the remainder of the plurality of targets are presented on the input/output unit within the specific presentation area.

In an example, the input/output unit is configured to detect brain activity of the user as they trace the trail between targets as they undertake the subset of the Trail Making Test. The determination of the performance level comprises utilization of the brain activity of the user as they trace the trail between targets as they undertake the subset of the Trail Making Test.

In a third aspect, there is provided a Trail Making Test method, comprising:
presenting by an input/output unit a calibration Trail Making Test to a user, wherein presentation of the calibration Trail Making Test comprises a plurality of targets being presented on the input/output unit;
undertaking by the user the calibration Trail Making Test, where the user is requested to trace a trail linking the plurality of targets in a specific order, and wherein a task difficulty level increases progressively with respect to the trail linking the plurality of targets in the specific order;
detecting by the input/output unit input from the user as they trace a trail between targets as they undertake at least some of the calibration Trail Making Test;
determining or selecting by a processing unit an evaluation Trail Making Test for the user comprising utilizing the detected input from the user; and
presenting by the input/output unit the evaluation Trail Making Test to the user, wherein presentation of the evaluation Trail Making Test comprises a plurality of targets being presented on the input/output unit, wherein to undertake the evaluation Trail Making Test the user is requested to trace a trail linking the plurality of targets in a specific order.

In a fourth aspect, there is provided a Trail Making Test method, comprising:
presenting by an input/output unit a subset of a Trail Making Test to a user, wherein presentation of the subset of the Trail Making Test comprises a subset of a plurality of targets being presented on the input/output unit;
undertaking by the user the subset of the Trail Making Test, where the user is requested to trace a trail linking the subset of the plurality of targets in a specific order;
detecting by the input/output unit input from the user as they trace a trail between targets as they undertake the subset of the Trail Making Test;
determining by the processing unit a performance level for the user, the determining comprising utilization of the detected input from the user;
wherein the method comprises the processing unit:
if the determined performance level matches a required performance level presenting the remainder of the Trail Making Test to the user, wherein presentation of the remainder of the Trail Making Test comprises a remainder of the plurality of targets being presented on the input/output unit, wherein to undertake the remainder of the Trail Making Test the user is requested to trace a trail linking the remainder of the plurality of targets in a specific order;
if the determined performance level does not match the required performance level carry out the following until the determined performance level matches the required performance level:
   adapting the Trail Making Test, wherein the adapting comprises utilizing the determined performance level;
   for the adapted Trail Making Test presenting a subset of the Trail Making Test to the user, wherein presentation of the subset of the Trail Making Test comprises a subset of a plurality of targets being presented on the input/output unit,
   for the adapted Trail Making Test undertaking by the user the subset of the Trail Making Test, where the user is requested to trace a trail linking the subset of the plurality of targets in a specific order;
   for the adapted Trail Making Test detecting by the input/output unit input from the user as they trace a trail between targets as they undertake the subset of the Trail Making Test;
   for the adapted Trail Making Test determining a performance level for the user, the determining comprising utilization of the detected input from the user; and
   for the adapted Trail Making Test when the determined performance level matches the required performance level presenting the remainder of the adapted Trail Making Test to the user.

In an aspect, there is provided a computer program element for controlling a system according to the first aspect, which when executed by a processor is configured to carry out the method of the third aspect.

In an aspect, there is provided a computer program element for controlling a system according to the second aspect, which when executed by a processor is configured to carry out the method of the fourth aspect.

Thus, according to aspects, there is provided computer program elements controlling one or more of the systems as previously described which, if the computer program element is executed by a processor, is adapted to perform the methods as previously described.

According to another aspect, there is provided computer readable media having stored the computer elements as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows the Trail Making Test in digital tablet-based technology as implemented by Digital Cognitive Diagnostics, Philips Healthcare;
Fig. 2 shows an illustration of the TMT-A on the left side, and an illustration of the TMT-B on the right side;
Fig. 3 shows an example of a Shape Trail Test with circles and rectangles (from; Zhao, Q. et al. (2013), Plos One, Vol. 8 (2). The Shape Trail Test: Application of aNew Variant of the Trail Making Test);
Fig. 4 shows an example of a Colours Trail Making Test in black & white (from: Kim, H.J. (2014), Plos One, Vol. 9 (2). Alternative Type of the Trail Making Test in Nonnative English-Speakers: The Trail Making Test-Black & White);
Fig. 5 shows an example of a new Trail Making Test system;
Fig. 6 shows an example of a new Trail Making Test system;
Fig. 7 shows an example of a new Trail Making Test method;
Fig. 8 shows an example of a new Trail Making Test method; and
Fig. 9 shows an example workflow associated with a new Trail Making Test.

### DETAILED DESCRIPTION OF EMBODIMENTS

The new technique provides an ability to provide a Trail Making Test such that the task is not too difficult or easy for a particular patient, participant or user. This is achieved by selecting or adapting the difficulty of the Trail Making Test, based on input from the patient, participant or user, in order to provide a Trail Making Test that is personalized for the patient, participant or user (referred to generally as user). Features of the Trail Making Test can be increased or decreased to adapt the difficulty of the trail, where these features include trail length, visual interference, level of symmetry, salience and number of quadrants or area of the available display unit used for presentation of the task, and as discussed above these features can be dynamically adapted based on input from the user to provide a Trail Making Test commensurate for the user. Therefore, the new technique ensures that a task is at least completed within a practical time (effectively to provide a limited time to fully complete a trail). Because the time to complete a trail depends on the ability of a subject, patient, participant or user, which is personal, it has been found that to optimize a limited time window the difficulty of the trail can be adapted according to personal ability of the user.

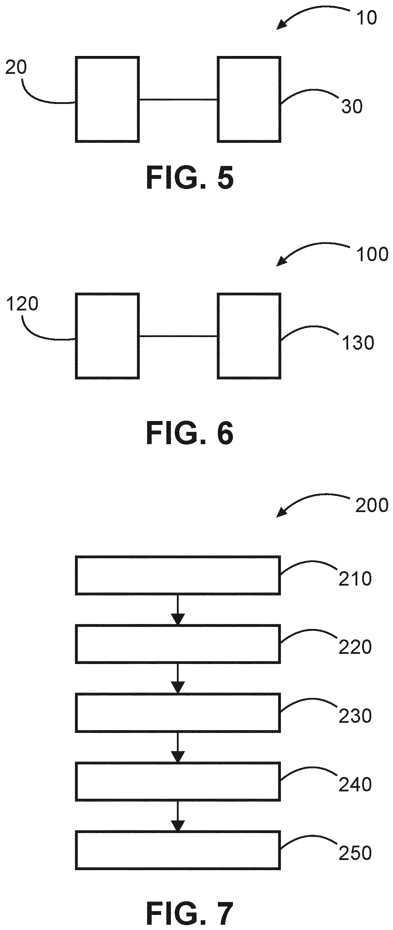

Fig. 5 shows an example of a Trail Making Test system 10. The TMT system comprises an input/output unit 20, and a processing unit 30. The input/output unit is configured to present a calibration Trail Making Test to a user. The presentation of the calibration Trail Making Test comprises a plurality of targets being presented on the input/output unit. To undertake the calibration Trail Making Test the user is requested to trace a trail linking the plurality of targets in a specific order, and a task difficulty level increases progressively with respect to the trail linking the plurality of targets in the specific order. The input/output unit is configured to detect input from the user as they trace a trail between targets as they undertake at least some of the calibration Trail Making Test. The processing unit is configured to determine or select an evaluation Trail Making Test for the user comprising utilization of the detected input from the user. The input/output unit is configured to present the evaluation Trail Making Test on the input/output unit. The presentation of the evaluation Trail Making Test comprises a plurality of targets being presented on the input/output unit, and to undertake the evaluation Trail Making Test the user is requested to trace a trail linking the plurality of targets in a specific order.

In an example, the targets comprise numbered icons.

In an example, the specific order of the targets comprises a numbered order of the icons.

In an example, the targets comprise lettered icons.

In an example, the specific order of the targets comprises an alphabetic order of the icons.

In an example, the targets comprise numbered icons and lettered icons.

In an example, the specific order of the targets comprises a numbered order of the numbered icons and an alphabetic order of the lettered icons.

In an example, the specific order of the targets comprises a numbered order of the numbered icons alternating with an alphabetic order of the lettered icons.

In an example, the input from the user as they trace a trail between targets as they undertake at least some of the calibration Trail Making Test comprises movement of a finger or stylus across a surface of the input/output unit. The input/output unit can for example comprise a computer tablet. It is also to be noted that input/output unit can mean more than one unit.

In an example, the processing unit is configured to determine a measure of the user's performance, and wherein the measure of the user's performance is based in part on a time taken for the user to complete the evaluation Trail Making Test.

According to an example, the processing unit is configured to utilize the detected input from the user to determine a task difficulty level for the user. The processing unit is configured to determine or select the evaluation Trail Making Test for the user comprising utilization of the determined task difficulty level for the user.

Thus, the system assesses how the user has completed or partially completed the calibration TMT and uses this to determine or select an appropriate TMT for the user to undertaken.

In an example, the processing unit is configured to determine a measure of the user's performance. The measure of the user's performance can be based in part on a time taken to complete a first evaluation Trail Making Test and a time taken to complete a second evaluation Trail Making Test having the same task difficulty level for the user as the first evaluation Trail Making Test.

In an example, the second evaluation Trail Making Test is identical to the first evaluation Trail Making Test.

In an example, the second evaluation Trail Making Test is different to the first evaluation Trail Making Test.

In other words, the system can carry out a longitudinal assessment of the user, where the user can periodically carry out the same TMT and the time taken to complete the TMT can be monitored to assess the user's performance. Also, the longitudinal assessment can be made for different TMTs having the same difficulty level for the user, to ensure that the user cannot use prior experience of a TMT to complete it faster than they would if they had not seen it before.

In an example, the processing unit is configured to determine a measure of the user's performance based in part on the task difficulty level determined for the user.

Thus, the task difficulty level determined for the evaluation TMT itself provides information on the user in addition to information obtained from the user completing the evaluation TMT. This also enables a longitudinal assessment to be made, because the determined task difficulty for the user, as the user uses the system periodically provides information on the user in addition to how the user completes the resultant evaluation TMT itself.

According to an example, the processing unit is configured to determine the task difficulty level for the user as the task difficulty level associated with adjacent targets of the trail linking the plurality of targets in the specific order, and the processing unit is configured to determine the adjacent targets for the determination of the task difficulty level. The determination of the adjacent targets for the determination of the task difficulty level can comprise a determination that the user exceeds a threshold time to trace a trail on the input/output unit linking the adjacent targets or a determination that the user fails to trace a trail between the adjacent targets within the threshold time.

To put this another way, as the task difficulty level increases progressively with respect to the trail linking the plurality of targets in the specific order, it is expected that it will take on average progressively longer for the user to trace a trail between adjacent targets of the trail. At some point in the trail the user will take a time that is commensurate with an average task difficulty level for a TMT that it is desired that the user undertakes for assessment, where for example it would be anticipated that the user should be able to complete this TMT in an appropriate timescale. The system can then select an appropriate TMT, from a number of different TMTs, that has the required task difficulty level, or the system can update or modify a standard TMT such that the task difficulty level of this standard TMT is changed to the required task difficulty level.

The threshold time can be associated with a time required to take an entire test at this or just below the difficulty level associated with the adjacent icons. Thus, if for an evaluation TMT 50 targets will be presented to the user and it is desired for the user to take about 5 minutes to undertake the evaluation TMT then on average there would be 6 seconds to be taken linking adjacent targets. This can be used to determine the threshold.

According to an example, the input/output unit is configured to detect brain activity of the user as they trace the trail between targets as they undertake at least some of the calibration Trail Making Test. The processing unit is configured to determine the task difficulty level for the user as the task difficulty level associated with adjacent targets of the trail linking the plurality of targets in the specific order. The processing unit is configured to determine the adjacent targets comprising utilization of the brain activity of the user as they undertake at least some of the calibration Trail Making Test.

Thus, as discussed above, the input/output unit can be more than one unit, and can be a tablet on which the TMT is presented to the user, and that could detect input from the user, and an input unit of the input/output unit can also be for example an EEG apparatus than detects brain activity.

In an example, the input from the user as they trace a trail between targets as they undertake at least some of the calibration Trail Making Test comprises the detected brain activity of the user.

In an example, the input from the user as they trace a trail between targets as they undertake at least some of the calibration Trail Making Test comprises movement of a finger or stylus across a surface of the input/output unit and the detected brain activity of the user.

Thus, the brain activity of the user can be used to determine when the user is finding undertaking the calibration TMT to be at a difficulty level that would be commensurate to apply for a whole evaluation TMT to be selected or determined. Alternatively, the brain activity can be utilized with information gleaned from how the user moves their finger or a stylus to trace a trail, for example to indicate that the person has been distracted by an extraneous event that has led to an increase in the time to connect targets or confirm that the user is indeed finding it difficult to connect targets.

According to an example, the processing unit is configured to select the evaluation Trail Making Test from a plurality of Trail Making Tests each having a different task difficulty level as a Trail Making Test of the plurality of Trail Making Tests that has a difficulty level that most closely matches the determined task difficulty level for the user.

According to an example, the processing unit is configured to determine the evaluation Trail Making Test by adaptation of a reference Trail Making Test to have a difficulty level that corresponds to the determined task difficulty level for the user.

In an example, adaptation of the reference Trail Making Test comprises one or more of: a change in distance between two or more of the targets of the plurality of targets in the specific order when presented on the input/output unit; a change in a number of targets not in sequence that lie within a threshold distance of the trail linking the plurality of targets when presented on the input/output unit; a change in the symmetry of the plurality of targets when presented on the input/output unit; a change in intensity level and/or colour between adjacent targets when presented on the input/output unit.

Fig. 6 shows an example of a Trail Making Test system 100. The TMT system comprises an input/output unit 120, and a processing unit 130. The input/output unit can be the same as the input/output unit 20. The input/output unit is configured to present a subset of a Trail Making Test to a user, and presentation of the subset of the Trail Making Test comprises a subset of a plurality of targets being presented on the input/output unit. To undertake the subset of the Trail Making Test the user is requested to trace a trail linking the subset of the plurality of targets in a specific order. The input/output unit is configured to detect input from the user as they trace a trail between targets as they undertake the subset of the Trail Making Test. The processing unit is configured to determine a performance level for the user, the determination comprising utilization of the detected input from the user.

The processing unit is configured to do both of the following, and for a particular situation does one or other of the following, and thus:

### Situation 1

if the determined performance level matches a required performance level present the remainder of the Trail Making Test to the user. The presentation of the remainder of the Trail Making Test comprises a remainder of the plurality of targets being presented on the input/output unit. To undertake the remainder of the Trail Making Test the user is requested to trace a trail linking the remainder of the plurality of targets in a specific order.

### Situation 2

if the determined performance level does not match the required performance level carry out the following until the determined performance level matches the required performance level:
adapt the Trail Making Test, wherein the adaptation comprises utilization of the determined performance level;
for the adapted Trail Making Test present of a subset of the Trail Making Test to the user, wherein presentation of the subset of the Trail Making Test comprises a subset of a plurality of targets being presented on the input/output unit, wherein to undertake the subset of the Trail Making Test the user is requested to trace a trail linking the subset of the plurality of targets in a specific order;
for the adapted Trail Making Test detect input from the user as they trace a trail between targets as they undertake the subset of the Trail Making Test;
for the adapted Trail Making Test determine a performance level for the user, the determination comprising utilization of the detected input from the user; and
for the adapted Trail Making Test when the determined performance level matches the required performance level present the remainder of the adapted Trail Making Test to the user.

In other words, a feedback loop system is provided where a user starts a first part of a TMT and if their performance is commensurate for the completion of a full test, the remainder of the test is presented to the user for completion. However, if the user is finding the TMT too easy and would complete a full TMT too quickly for example, a new set of targets is presented to the user that should be easier for the user to complete and if user is finding the TMT too difficult and would complete a full TMT a new set of targets is provided to the user that should be more difficult for the user to complete. This process iterates until the user undertakes the subset of a finally adapted TMT that is commensurate with the user carrying out a full TMT in a reasonable time and they are then presented with the remainder of this TMT to complete.

The user can be presented with a completely new subset of adapted targets, for example, the user could link 10 targets by a trail, then be presented with a new 10 targets to link at a different difficultly level, until the user reaches the required performance level and the remainder of the targets are presented.

In an example, the subset of the Trail Making Test has 50% of the targets of the Trail Making Test.

In an example, the subset of the Trail Making Test has 40% of the targets of the Trail Making Test.

In an example, the subset of the Trail Making Test has 30% of the targets of the Trail Making Test.

In an example, the subset of the Trail Making Test has 20% of the targets of the Trail Making Test.

In an example, the subset of the Trail Making Test has 10% of the targets of the Trail Making Test.

In an example, the subset of the Trail Making Test has less than 10% of the targets of the Trail Making Test.

In an example, the targets comprise numbered icons.

In an example, the specific order of the targets comprises a numbered order of the icons.

In an example, the targets comprise lettered icons.

In an example, the specific order of the targets comprises an alphabetic order of the icons.

In an example, the targets comprise numbered icons and lettered icons.

In an example, the specific order of the targets comprises a numbered order of the numbered icons and an alphabetic order of the lettered icons.

In an example, the specific order of the targets comprises a numbered order of the numbered icons alternating with an alphabetic order of the lettered icons.

In an example, the input from the user as they trace a trail between targets as they undertake at least some of the calibration Trail Making Test comprises movement of a finger or stylus across a surface of the input/output unit.

In an example, the processing unit is configured to determine a measure of the user's performance. The measure of the user's performance can be based in part on a time taken for the user to complete the subset of the Trail Making Test for which the determined performance level for the user matches the required performance level.

In an example, the processing unit is configured to determine a measure of the user's performance. The measure of the user's performance can be based in part on a time taken for the user to complete the Trail Making Test for which the determined performance level for the user matches the required performance level.

Thus, even though the final TMT should result in an ideal time or ideal time window for completion of the TMT, the patient taking the same TMT at different times can exhibit a change in the time to complete the TMT and this can be used to monitor changes in the performance of the user.

In an example, the processing unit is configured to determine a measure of the user's performance. The measure of the user's performance can be based in part on a time taken to complete a first Trail Making Test for which the determined performance level for the user matches the required performance level and a time taken to complete a second Trail Making Test having the same task difficulty level for the user as the first evaluation Trail Making Test.

In an example, the second Trail Making Test is identical to the first evaluation Trail Making Test.

In an example, the second evaluation Trail Making Test is different to the first evaluation Trail Making Test.

Thus, the system can carry out a longitudinal assessment of the user, where the user can periodically carry out the same TMT and the time taken to complete the TMT can be monitored to assess the user's performance. Also, the longitudinal assessment can be made for different TMTs having the same difficulty level for the user, to ensure that the user cannot use prior experience of a TMT to complete it faster than they would if they had not seen it before.

In an example, the processing unit is configured to determine a measure of the user's performance based in part on the difficulty level of the TMT for which the determined performance level for the user matches the required performance level.

In this manner, the actual difficulty level of a TMT determined for the user can provide information of the user. Thus, if the user periodically interacts with the system even though a final TMT determined for the user should be able to be completed within a set time or set time window, the difficulty level for the user could change over time, for example the difficulty level could increase showing an increase in cognitive capability, or the difficulty level could decrease showing a decrease in cognitive capability. Also, a single difficulty level for a TMT determined for the patient can be compared against that for other similar users to provide a comparative analysis of cognitive ability simply on the basis of the difficulty level of the TMT that the patient should be able to carry out in a set time.

According to an example, the processing unit is configured to utilize the detected input from the user as they trace the trail between targets as they undertake the subset of the Trail Making Test to determine a time for the user to complete the subset of the Trail Making Test. The determined performance level can comprise the time for the user to complete the subset of the Trail Making Test. The processing unit is configured to determine that the determined performance level matches the required performance level comprising a determination that the time for the user to complete the subset of the Trail Making Test is within a threshold time of an ideal time for the user to complete a subset of a Trail Making Test.

In other words, it can be that a medical practitioner desires a patient to complete a TMT in around 5 minutes. If the patient uses the system and from the first targets it is clear that the patient would require 20 minutes to complete a full test, the TMT is adapted to be easier and the patient starts a new TMT with the new targets, and this iterates until it is indicated that the patient would complete the full TMT for which they are interacting with a subset in 5 minutes, and the patient is provided with the rest of this TMT to finish.

In an example, the processing unit is configured to utilize the detected input from the user as they trace the trail between targets as they undertake the subset of the Trail Making Test to determine an estimated time for the user to complete the Trail Making Test. The determined performance level can comprise the estimated time for the user to complete the Trail Making Test. The processing unit is configured to determine that the determined performance level matches the required performance level comprising a determination that the estimated time for the user to complete the Trail Making Test is within a threshold time of an ideal time for the user to complete a Trail Making Test.

According to an example, the processing unit is configured to adapt the Trail Making Test to increase a task difficulty level of the Trail Making Test if the time for the user to complete the subset of the Trail Making Test is below the ideal time for the user to complete a subset of a Trail Making Test by more than the threshold time. The processing unit is configured to adapt the Trail Making Test to decrease a task difficulty level of the Trail Making Test if the time for the user to complete the subset of the Trail Making Test is above the ideal time for the user to complete a subset of a Trail Making Test by more than the threshold time.

In an example, the processing unit is configured to adapt the Trail Making Test to increase a task difficulty level of the Trail Making Test if the estimated time for the user to complete the Trail Making Test is below the ideal time for the user to complete a Trail Making Test by more than the threshold time. The processing unit is configured to adapt the Trail Making Test to decrease a task difficulty level of the Trail Making Test if the estimated time for the user to complete the Trail Making Test is above the ideal time for the user to complete a Trail Making Test by more than the threshold time.

According to an example, adaptation of the Trail Making Test comprises one or more of: a change in distance between two or more of the targets of the plurality of targets in the specific order when presented on the input/output unit; a change in a number of targets not in sequence that lie within a threshold distance of the trail linking the plurality of targets when presented on the input/output unit; a change in the symmetry of the plurality of targets when presented on the input/output unit; a change in intensity level and/or colour between adjacent targets when presented on the input/output unit.

In an example, adaptation of the Trail Making Test to increase the task difficulty level comprises one or more of: an increase in distance between two or more of the targets of the plurality of targets in the specific order when presented on the input/output unit; an increase in the number of targets not in sequence that lie within a threshold distance of the trail linking the plurality of targets when presented on the input/output unit; a decrease in the symmetry of the plurality of targets when presented on the input/output unit; a change in intensity level and/or colour between adjacent targets when presented on the input/output unit.

In an example, adaptation of the Trail Making Test to decrease the task difficulty level comprises one or more of: a decrease in distance between two or more of the targets of the plurality of targets in the specific order when presented on the input/output unit; a decrease in the number of targets not in sequence that lie within a threshold distance of the trail linking the plurality of targets when presented on the input/output unit; an increase in the symmetry of the plurality of targets when presented on the input/output unit; a change in intensity level and/or colour between adjacent targets when presented on the input/output unit; an identification of the first target in the sequence.

According to an example, the processing unit is configured to determine from the input from the user as they trace a trail between targets as they undertake the subset of the Trail Making Test that the user has a deficiency with respect to a specific presentation area of the input/output unit less than the overall presentation area of the input/output unit. In this case, for the adapted Trail Making Test the processing unit is configured such that none of the subset of the plurality of targets or the remainder of the plurality of targets are presented on the input/output unit within the specific presentation area.

According to an example, the input/output unit is configured to detect brain activity of the user as they trace the trail between targets as they undertake the subset of the Trail Making Test. The determination of the performance level can comprise utilization of the brain activity of the user as they trace the trail between targets as they undertake the subset of the Trail Making Test.

In the case, the input/output unit can have an input unit part in the form of an EEG apparatus. The input/output unit can also have for example a tablet for presentation of the TMT, and that can if necessary detect how the user traces a trail on the tablet's screen

In an example, the input from the user as they trace a trail between targets as they undertake the subset of the Trail Making Test comprises the detected brain activity of the user.

In an example, the input from the user as they trace a trail between targets as they undertake the subset of the Trail Making Test comprises movement of a finger or stylus across a surface of the input/output unit and the detected brain activity of the user.

In this manner, the brain activity of the user can be used to determine when the user is finding undertaking the subset of the TMT to be at a difficulty level that would be commensurate to apply for a whole TMT to be undertaken by the user, or to determine that the user is finding undertaking the subset of the TMT too easy or difficult enabling a TMT to be adapted accordingly as part of an iterative process, Alternatively, the brain activity can be utilized with information gleaned from how the user moves their finger or a stylus to trace a trail, for example to indicate that the person has been distracted by an extraneous event that has led to an increase in the time to connect targets or confirm that the user is indeed finding it difficult/easy to connect targets.

Fig. 7 shows an example of a Trail Making Test method 200, comprising:
presenting 210 by an input/output unit a calibration Trail Making Test to a user, wherein presentation of the calibration Trail Making Test comprises a plurality of targets being presented on the input/output unit;
undertaking 220 by the user the calibration Trail Making Test, where the user is requested to trace a trail linking the plurality of targets in a specific order, and wherein a task difficulty level increases progressively with respect to the trail linking the plurality of targets in the specific order;
detecting 230 by the input/output unit input from the user as they trace a trail between targets as they undertake at least some of the calibration Trail Making Test;
determining or selecting 240 by a processing unit an evaluation Trail Making Test for the user comprising utilizing the detected input from the user; and
presenting 250 by the input/output unit the evaluation Trail Making Test to the user, wherein presentation of the evaluation Trail Making Test comprises a plurality of targets being presented on the input/output unit, wherein to undertake the evaluation Trail Making Test the user is requested to trace a trail linking the plurality of targets in a specific order.

In an example, the targets comprise numbered icons.

In an example, the specific order of the targets comprises a numbered order of the icons.

In an example, the targets comprise lettered icons.

In an example, the specific order of the targets comprises an alphabetic order of the icons.

In an example, the targets comprise numbered icons and lettered icons.

In an example, the specific order of the targets comprises a numbered order of the numbered icons and an alphabetic order of the lettered icons.

In an example, the specific order of the targets comprises a numbered order of the numbered icons alternating with an alphabetic order of the lettered icons.

In an example, the input from the user as they trace a trail between targets as they undertake at least some of the calibration Trail Making Test comprises movement of a finger or stylus across a surface of the input/output unit.

In an example, the processing unit is configured to determine a measure of the user's performance, and wherein the measure of the user's performance is based in part on a time taken for the user to complete the evaluation Trail Making Test.

In an example, the method comprises utilizing by the processing unit the detected input from the user to determine a task difficulty level for the user. The determining or selecting by the processing unit the evaluation Trail Making Test for the user can then comprise utilizing the determined task difficulty level for the user.

In an example, method comprises determining by the processing unit a measure of the user's performance. The measure of the user's performance can be based in part on a time taken to complete a first evaluation Trail Making Test and a time taken to complete a second evaluation Trail Making Test having the same task difficulty level for the user as the first evaluation Trail Making Test.

In an example, the second evaluation Trail Making Test is identical to the first evaluation Trail Making Test.

In an example, the second evaluation Trail Making Test is different to the first evaluation Trail Making Test.

In an example, the method comprises determining by the processing unit a measure of the user's performance based in part on the task difficulty level determined for the user.

In an example, the method comprises determining by the processing unit the task difficulty level for the user as the task difficulty level associated with adjacent targets of the trail linking the plurality of targets in the specific order. The method comprises determining by the processing unit the adjacent targets for the determination of the task difficulty level. The determining the adjacent targets for the determination of the task difficulty level can comprise determining that the user exceeds a threshold time to trace a trail on the input/output unit linking the adjacent targets or a determining that the user fails to trace a trail between the adjacent targets within the threshold time.

In an example, the method comprises detecting by the input/output unit brain activity of the user as they trace the trail between targets as they undertake at least some of the calibration Trail Making Test. The method comprises determining by the processing unit the task difficulty level for the user as the task difficulty level associated with adjacent targets of the trail linking the plurality of targets in the specific order. The method comprises determining by the processing unit the adjacent targets comprising utilizing the brain activity of the user as they undertake at least some of the calibration Trail Making Test.

In an example, the input from the user as they trace a trail between targets as they undertake at least some of the calibration Trail Making Test comprises the detected brain activity of the user.

In an example, the input from the user as they trace a trail between targets as they undertake at least some of the calibration Trail Making Test comprises movement of a finger or stylus across a surface of the input/output unit and the detected brain activity of the user.

In an example, method comprises selecting by the processing unit the evaluation Trail Making Test from a plurality of Trail Making Tests, each having a different task difficulty level, as a Trail Making Test of the plurality of Trail Making Tests that has a difficulty level that most closely matches the determined task difficulty level for the user.

In an example, the method comprises determining by the processing unit the evaluation Trail Making Test by adapting a reference Trail Making Test to have a difficulty level that corresponds to the determined task difficulty level for the user.

In an example, adaptation of the reference Trail Making Test comprises one or more of: a change in distance between two or more of the targets of the plurality of targets in the specific order when presented on the input/output unit; a change in a number of targets not in sequence that lie within a threshold distance of the trail linking the plurality of targets when presented on the input/output unit; a change in the symmetry of the plurality of targets when presented on the input/output unit; a change in intensity level and/or colour between adjacent targets when presented on the input/output unit.

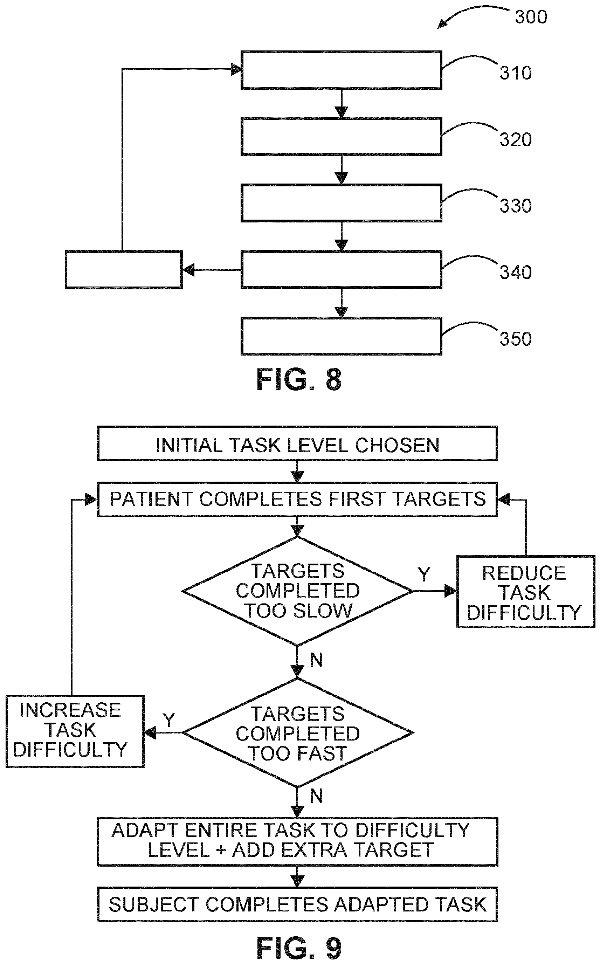

Fig. 8 shows an example of a Trail Making Test method 300, comprising:
presenting 310 by an input/output unit a subset of a Trail Making Test to a user, wherein presentation of the subset of the Trail Making Test comprises a subset of a plurality of targets being presented on the input/output unit;
undertaking 320 by the user the subset of the Trail Making Test, where the user is requested to trace a trail linking the subset of the plurality of targets in a specific order;
detecting 330 by the input/output unit input from the user as they trace a trail between targets as they undertake the subset of the Trail Making Test;
determining 340 by the processing unit a performance level for the user, the determining comprising utilization of the detected input from the user;
wherein the method comprises the processing unit carry out the following:

### Situation 1

if the determined performance level matches a required performance level presenting 350 the remainder of the Trail Making Test to the user, wherein presentation of the remainder of the Trail Making Test comprises a remainder of the plurality of targets being presented on the input/output unit, wherein to undertake the remainder of the Trail Making Test the user is requested to trace a trail linking the remainder of the plurality of targets in a specific order;

### Situation 2

if the determined performance level does not match the required performance level carry out the following until the determined performance level matches the required performance level: adapting 360a-n the Trail Making Test, wherein the adapting comprises utilizing the determined performance level;
for the adapted Trail Making Test presenting 310a-n a subset of the Trail Making Test to the user, wherein presentation of the subset of the Trail Making Test comprises a subset of a plurality of targets being presented on the input/output unit,
for the adapted Trail Making Test undertaking 320a-n by the user the subset of the Trail Making Test, where the user is requested to trace a trail linking the subset of the plurality of targets in a specific order;
for the adapted Trail Making Test detecting 330a-n by the input/output unit input from the user as they trace a trail between targets as they undertake the subset of the Trail Making Test;
for the adapted Trail Making Test determining 340a-n a performance level for the user, the determining comprising utilization of the detected input from the user; and
for the adapted Trail Making Test when the determined performance level matches the required performance level presenting 350 the remainder of the adapted Trail Making Test to the user.

Here the numerals a-n indicate that these method steps can be iterated until completion.

In an example, the subset of the Trail Making Test has 50% of the targets of the Trail Making Test.

In an example, the subset of the Trail Making Test has 40% of the targets of the Trail Making Test.

In an example, the subset of the Trail Making Test has 30% of the targets of the Trail Making Test.

In an example, the subset of the Trail Making Test has 20% of the targets of the Trail Making Test.

In an example, the subset of the Trail Making Test has 10% of the targets of the Trail Making Test.

In an example, the subset of the Trail Making Test has less than 10% of the targets of the Trail Making Test.

In an example, the targets comprise numbered icons.

In an example, the specific order of the targets comprises a numbered order of the icons.

In an example, the targets comprise lettered icons.

In an example, the specific order of the targets comprises an alphabetic order of the icons.

In an example, the targets comprise numbered icons and lettered icons.

In an example, the specific order of the targets comprises a numbered order of the numbered icons and an alphabetic order of the lettered icons.

In an example, the specific order of the targets comprises a numbered order of the numbered icons alternating with an alphabetic order of the lettered icons.

In an example, the input from the user as they trace a trail between targets as they undertake at least some of the calibration Trail Making Test comprises movement of a finger or stylus across a surface of the input/output unit.

In an example, the method comprises determining by the processing unit a measure of the user's performance. The measure of the user's performance can be based in part on a time taken for the user to complete the subset of the Trail Making Test for which the determined performance level for the user matches the required performance level.

In an example, the method comprises determining by the processing unit a measure of the user's performance. The measure of the user's performance can be based in part on a time taken for the user to complete the Trail Making Test for which the determined performance level for the user matches the required performance level.

In an example, the method comprises determining by the processing unit a measure of the user's performance. The measure of the user's performance can be based in part on a time taken to complete a first Trail Making Test for which the determined performance level for the user matches the required performance level and a time taken to complete a second Trail Making Test having the same task difficulty level for the user as the first evaluation Trail Making Test.

In an example, the second Trail Making Test is identical to the first evaluation Trail Making Test.

In an example, the second evaluation Trail Making Test is different to the first evaluation Trail Making Test.

In an example, the method comprises determining by the processing unit a measure of the user's performance based in part on a difficulty level of the TMT for which the determined performance level for the user matches the required performance level.

In an example, method comprises utilizing by the processing unit the detected input from the user as they trace the trail between targets as they undertake the subset of the Trail Making Test to determine a time for the user to complete the subset of the Trail Making Test. The determined performance level can comprise the time for the user to complete the subset of the Trail Making Test. The method comprises determining by the processing unit that the determined performance level matches the required performance level comprising determining that the time for the user to complete the subset of the Trail Making Test is within a threshold time of an ideal time for the user to complete a subset of a Trail Making Test.

In an example, the method comprises utilizing by the processing unit the detected input from the user as they trace the trail between targets as they undertake the subset of the Trail Making Test to determine an estimated time for the user to complete the Trail Making Test. The determined performance level can comprise the estimated time for the user to complete the Trail Making Test. The method comprises determining by the processing unit that the determined performance level matches the required performance level comprising determining that the estimated time for the user to complete the Trail Making Test is within a threshold time of an ideal time for the user to complete a Trail Making Test.

In an example, the method comprises adapting by the processing unit the Trail Making Test to increase a task difficulty level of the Trail Making Test if the time for the user to complete the subset of the Trail Making Test is below the ideal time for the user to complete a subset of a Trail Making Test by more than the threshold time. The method comprises adapting by the processing unit the Trail Making Test to decrease a task difficulty level of the Trail Making Test if the time for the user to complete the subset of the Trail Making Test is above the ideal time for the user to complete a subset of a Trail Making Test by more than the threshold time.

In an example, the method comprises adapting by the processing unit the Trail Making Test to increase a task difficulty level of the Trail Making Test if the estimated time for the user to complete the Trail Making Test is below the ideal time for the user to complete a Trail Making Test by more than the threshold time. The method comprises adapting by the processing unit the Trail Making Test to decrease a task difficulty level of the Trail Making Test if the estimated time for the user to complete the Trail Making Test is above the ideal time for the user to complete a Trail Making Test by more than the threshold time.

In an example, adapting the Trail Making Test comprises one or more of: a change in distance between two or more of the targets of the plurality of targets in the specific order when presented on the input/output unit; a change in a number of targets not in sequence that lie within a threshold distance of the trail linking the plurality of targets when presented on the input/output unit; a change in the symmetry of the plurality of targets when presented on the input/output unit; a change in intensity level and/or colour between adjacent targets when presented on the input/output unit.

In an example, adapting the Trail Making Test to increase the task difficulty level comprises one or more of: an increase in distance between two or more of the targets of the plurality of targets in the specific order when presented on the input/output unit; an increase in the number of targets not in sequence that lie within a threshold distance of the trail linking the plurality of targets when presented on the input/output unit; a decrease in the symmetry of the plurality of targets when presented on the input/output unit; a change in intensity level and/or colour between adjacent targets when presented on the input/output unit.

In an example, adapting the Trail Making Test to decrease the task difficulty level comprises one or more of: a decrease in distance between two or more of the targets of the plurality of targets in the specific order when presented on the input/output unit; a decrease in the number of targets not in sequence that lie within a threshold distance of the trail linking the plurality of targets when presented on the input/output unit; an increase in the symmetry of the plurality of targets when presented on the input/output unit; a change in intensity level and/or colour between adjacent targets when presented on the input/output unit; an identification of the first target in the sequence.

In an example, the method comprises determining by the processing unit from the input from the user as they trace a trail between targets as they undertake the subset of the Trail Making Test that the user has a deficiency with respect to a specific presentation area of the input/output unit less than the overall presentation area of the input/output unit. For the adapted Trail Making Test none of the subset of the plurality of targets or the remainder of the plurality of targets are presented on the input/output unit within the specific presentation area.

In an example, the method comprises detecting by the input/output unit brain activity of the user as they trace the trail between targets as they undertake the subset of the Trail Making Test. The determining the performance level can comprise utilizing the brain activity of the user as they trace the trail between targets as they undertake the subset of the Trail Making Test.

In an example, the input from the user as they trace a trail between targets as they undertake the subset of the Trail Making Test comprises the detected brain activity of the user.

In an example, the input from the user as they trace a trail between targets as they undertake the subset of the Trail Making Test comprises movement of a finger or stylus across a surface of the input/output unit and the detected brain activity of the user.

The new Trail Making Test systems and Trail Making Test methods are now described in further specific detail, where reference is made to Fig. 9.

As detailed above, the new technique involves adapting the difficulty of the task (to be completed in the TMT) in such a manner to ensure the task can be performed in a limited time window and the subject's or user's performance quantified. In case the user, such as a patient, is struggling with the task and would take longer than a predefined period of time to complete the task (or indeed fail to complete the task at all) the task will be adapted to become easier.

Two approaches are considered for assessing if the task requires adaptation

In a first approach a separate calibration task is performed to assess the subject's or user's performance. Such a calibration task can for example be of any known trail making task providing the task difficulty increases as the task proceeds. By monitoring when the subject/user starts to struggle (e.g. slowing down, hovering, etc) it is possible to assess a suitable level of task difficulty, and it is also able to determine when the subject/user takes a time linking targets of the TMT in a time frame that would be commensurate with the time desired for the subject/user to carry out a full TMT, that would then be analysed. The TMT with an increasing difficulty level can be formed from a combination of different TMTs having different difficulty levels.

In a second, exemplified in the workflow shown in Fig. 9, an adaptive feedback system is provided. The user is shown X % of the targets of a TMT. X will be generally less than 50% of the targets, and can be less than 40%, less than 30%, less than 20%, less than 10%. Thus, a TMT could have 50 targets to be linked in some order when presented on a monitor. The user is however shown 5 of these targets, and uses a stylus to trace a trail linking the targets in a required order, where the monitor can detect the movement of the stylus. If the user is progressing too quickly, and a full TMT would be completed too quickly, the TMT is adapted and the first 5 targets of the adapted TMT are presented to user. The user again traces a trail linking these targets, and this adaptation of the TMT and presentation of the first 5 targets and tracing a trail by the user iterates until the user takes a time for the first 5 targets that would indicate that the full TMT would be completely in an appropriate time, and then the remainder of the TMT targets is presented to the user, thus 45 more targets, in addition to the 5 that the user has just completed. Rather than adapt a full TMT at each iteration, only the first 5 targets of a TMT need to be adapted until the iterative process suitable performance for the user, and then the remainder of the TMT can then be generated and presented to the user.

A controller, processing unit, configured to dynamically adjust one or more parameters of the task (e.g. trail length, visual interference, symmetry, salience, #quadrants) in order to ensure the task is operating in its sensitive range, such that the difficulty level is increased or decreased as required to iterate towards an optimum TMT task for the user.

The following provides more details on the features that can be adapted to change the difficulty of the task.
Trail Length: distance between targets, measured in centimeters
Visual Interference: X = number of targets that lie within a set distance (for example 3 centimeters) of the path between each target
Symmetry: cumulative distance for all mirrored targets. The targets at one side of the trail are mirrored to the other side across an imaginary midline of the trail. Then, the distance is calculated between the mirrored targets and the actual targets of the actual trail. The cumulative distance for all mirrored targets provides an estimate of the symmetry of the trail.

Quadrants/presentation area: relates to a good visual field or mitigating a neglected visual field. In special cases, e.g. for patients that suffered a stroke, neglect is a common symptom where a part of the visual field is neglected. Therefore, the TMT can be adapted for these patients such that a trail falls within the active visual field of the patient, or the attention of the patient can be triggered in a non-active part of the visual field by a flickering target. This option provides the possibility to have an alternative test for the Clock Drawing Test (see: Chen, P. & Goedert, K.M. (2012). Clock drawing in spatial neglect: A comprehensive analysis of clock perimeter, placement and accuracy. J Neuropsychol. 2012 September; 6(2): 270-289) which is well known for its diagnostic purpose in neglect patients.

Salience: this relates to salience measure of a target, which could be either flicker frequency or intensity, or size, or colour of the target. The noticeability of a target can be varied by e.g. making succeeding targets blink or flicker. And then, the flicker frequency and flicker intensity can be varied on a continuous scale from no flicker to high-frequency flicker with high light intensity. Next to the flicker, also colour can be used as a variable to vary. Succeeding targets can e.g. be highlighted in the same or a different colour to vary the salience and adapt the difficulty of the trail.

In a specific embodiment, if the difficulty level is to be adapted, the controller starts with the first feature, which is the trail length, and a TMT is adapted with respect to increased/decreased distances between targets as required. Then, the performance of the subject/user is checked again on for example the first 5 targets of the adapted TMT, and if the subject/user can finish the trail within a desired time limit, then this is the right difficulty level. However, if the subject/user takes more time to connect the targets that again indicates that the desired time for a complete TMT would not be met, then the controller adapts the second feature from the feature table, the visual interference parameter to make the adapted TMT more or less difficult as required. Again, the subject/user undertakes the first subject of the adapted TMT and is assessed. Ig necessary the other features are adapted in turn etc until a TMT is generated that has a difficulty level matched to the subject/user, where they are expected to complete the complete TMT in a desired time.

It can be determined that the subject/user has particular neuropsychological problems, and the orders ion which features are adapted can account for this. For example, for a user with a neglect problem, with is an attention deficit, a feature determined to be adapted first can be salience, for example to flickering the targets, or by changing the colour of the targets, and even flashing the first target. For a user, who have left-right asymmetry in their vision, symmetry can be a feature to be adapted first. Consider a patient who, after a stroke, is solely able to perceive targets in the left visual hemifield. For such a patient it would make sense to position the midline a predefined distance to the left. In principle the midline (in fact the mirror-symmetry line) can also be a curved line. For a user targets for the subset of the TMT the patient is to perform can be spaced over a whole display area, for example over the four quadrants of the display area, and it can be determined that the user has difficult with targets presented in one or more quadrants. Thus, the TMT can be adapted to not present targets in these quadrants, where the user has a deficiency, and thus the TMT can be adapted appropriately even when there is no prior knowledge of a deficiency of the user.

Thus, for example because neglect is an attentional problem, increasing the feature Salience will track more attention to the targets, and eventually it can be desired to track attention to the neglected part of the visual field. This can be done for example by the consecutively blinking / flickering of succeeding targets, which may be laying in the left part of the visual field that is being neglected. If attention is also available for the neglected visual field, then the trail is easier to complete. After adapting the salience of the targets of the trail, the performance is measured again and the process starts over. Preferably, the measurements approach a time associated with a desired time for the complete TMT. As detailed above, the feature values detailed above are dynamically adapted. A controller adapts the trail, and to prevent that the system starts oscillating, the average feature value over a specific (sliding) time block can be taken.

If the option of increasing Salience is not successful, we can shift the adaption of the trail to less than 4 quadrants, depending on the neglected part of the visual field. In case of neglecting the left part of the visual field, we can e.g. present targets only in the right part of the visual field. This makes the completion of the trail easier.

Thus, the user's performance is measured in space (x,y coordinates) and in time (total path time, time to connect a target). The task (connecting a trail of the targets) provides a quantitative result if the difficulty level does not provide a ceiling effect or a floor effect, and it is preferred that a subject completes the trail in a reasonable amount of time. If the completion of an evaluation part of a TMT indicates that the user would complete the full TMT too quickly/slowly, then, the task difficulty of the TMT is adapted by changing one of the exemplary variables such as trail length, visual interference, symmetry and number of quadrants, and this iterates until a personalized TMT for the user is generated.

The personalization of the TMT for a user enables a comparison of longitudinal results (a comparison of results over time) between sessions of trials to be made. A prerequisite for such longitudinal comparisons is that the trial can be quantified, and the new technique enables personalisation of the task leads to the situation where task are expected to be completed within a time window and quantification of task performance is made possible. Thus, once the level of difficulty for a TMT has been established required to ensure the trial is in the sensitive range where quantification is optimised, the following can be undertaken:
Compare the results of the present trial with results of earlier trials measured at the same level of difficulty. For this reason, it is advantageous if there are only certain discrete levels of difficulty defined.
Establish relative scores between trials at different difficulty levels by applying a calibration factor (for example from population data of results of individuals carrying out all the different levels of the trail)

The actual difficulty level of a TMT generated for a user provides an outcome measure for the user itself, and this can relate to an overall difficulty index or relate to how the features of trail length, visual interference, symmetry, salience, #quadrants have been adapted for this particular user in generating the personalized TMT for this user. Thus, feature variables are used to provide an additional outcome measure. An analysis of the proposed variables (e.g. trail length, visual inference, ...) can provide a difficulty level of a particular version of the TMT. The new outcome measure can involve comparing the difficulty of the TMT version to the time the patient needs to complete the task. This outcome measure would bypass the use of norm tables (which must be maintained in order to remain accurate as the general population is getting more capable over time). It would also allow for a more precise scoring, instead of 'healthy range' vs 'impaired range'. It enables a comparison between multiple tests without a norm table.

Next to the performance on the task, another personal input variable that provides information is the brain activation. The brain activation can be used to indicate where the specific problem is by using the location of the brain activation and the intensity of the brain activation. Also, measuring brain activity can provide an indication of task difficulty. This can be utilized alone or in combination with the discussions above in the system to generate the personalized TMT.

In another exemplary embodiment, a computer program or computer program element is provided that is characterized by being configured to execute the method steps of any of the methods according to one of the preceding embodiments, on an appropriate apparatus or system.

The computer program element might therefore be stored on a computer unit, which might also be part of an embodiment. This computing unit may be configured to perform or induce performing of the steps of the method described above. Moreover, it may be configured to operate the components of the above described system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A Trail Making Test system (10), comprising:
- an input/output unit (20); and
- a processing unit (30); and
wherein the input/output unit is configured to present a calibration Trail Making Test to a user, wherein presentation of the calibration Trail Making Test comprises a plurality of targets being presented on the input/output unit, wherein to undertake the calibration Trail Making Test the user is requested to trace a trail linking the plurality of targets in a specific order, and wherein a task difficulty level increases progressively with respect to the trail linking the plurality of targets in the specific order;
wherein the input/output unit is configured to detect input from the user as they trace a trail between targets as they undertake at least some of the calibration Trail Making Test;
wherein the processing unit is configured to determine or select an evaluation Trail Making Test for the user comprising utilization of the detected input from the user; and
wherein the input/output unit is configured to present the evaluation Trail Making Test on the input/output unit, wherein presentation of the evaluation Trail Making Test comprises a plurality of targets being presented on the input/output unit, wherein to undertake the evaluation Trail Making Test the user is requested to trace a trail linking the plurality of targets in a specific order.

2. System according to claim 1, wherein the processing unit is configured to utilize the detected input from the user to determine a task difficulty level for the user, and wherein the processing unit is configured to determine or select the evaluation Trail Making Test for the user comprising utilization of the determined task difficulty level for the user.

3. System according to claim 2, wherein the processing unit is configured to determine the task difficulty level for the user as the task difficulty level associated with adjacent targets of the trail linking the plurality of targets in the specific order, wherein the processing unit is configured to determine the adjacent targets for the determination of the task difficulty level, and wherein the determination of the adjacent targets for the determination of the task difficulty level comprises a determination that the user exceeds a threshold time to trace a trail on the input/output unit linking the adjacent targets or a determination that the user fails to trace a trail between the adjacent targets within the threshold time.

4. System according to any of claims 2-3, wherein the input/output unit is configured to detect brain activity of the user as they trace the trail between targets as they undertake at least some of the calibration Trail Making Test, wherein the processing unit is configured to determine the task difficulty level for the user as the task difficulty level associated with adjacent targets of the trail linking the plurality of targets in the specific order, wherein the processing unit is configured to determine the adjacent targets comprising utilization of the brain activity of the user as they undertake at least some of the calibration Trail Making Test.

5. System according to any of claims 2-4, wherein the processing unit is configured to select the evaluation Trail Making Test from a plurality of Trail Making Tests each having a different task difficulty level as a Trail Making Test of the plurality of Trail Making Tests that has a difficulty level that most closely matches the determined task difficulty level for the user.

6. System according to any of claims 2-4, wherein the processing unit is configured to determine the evaluation Trail Making Test by adaptation of a reference Trail Making Test to have a difficulty level that corresponds to the determined task difficulty level for the user.

7. A Trail Making Test system (100), comprising:
- an input/output unit (120); and
- a processing unit (130); and
wherein the input/output unit is configured to present a subset of a Trail Making Test to a user, wherein presentation of the subset of the Trail Making Test comprises a subset of a plurality of targets being presented on the input/output unit, wherein to undertake the subset of the Trail Making Test the user is requested to trace a trail linking the subset of the plurality of targets in a specific order;
wherein the input/output unit is configured to detect input from the user as they trace a trail between targets as they undertake the subset of the Trail Making Test;
wherein the processing unit is configured to determine a performance level for the user, the determination comprising utilization of the detected input from the user;
wherein the processing unit is configured to:
if the determined performance level matches a required performance level present the remainder of the Trail Making Test to the user, wherein presentation of the remainder of the Trail Making Test comprises a remainder of the plurality of targets being presented on the input/output unit, wherein to undertake the remainder of the Trail Making Test the user is requested to trace a trail linking the remainder of the plurality of targets in a specific order;
if the determined performance level does not match the required performance level carry out the following until the determined performance level matches the required performance level: adapt the Trail Making Test, wherein the adaptation comprises utilization of the determined performance level;
for the adapted Trail Making Test present of a subset of the Trail Making Test to the user, wherein presentation of the subset of the Trail Making Test comprises a subset of a plurality of targets being presented on the input/output unit, wherein to undertake the subset of the Trail Making Test the user is requested to trace a trail linking the subset of the plurality of targets in a specific order;
for the adapted Trail Making Test detect input from the user as they trace a trail between targets as they undertake the subset of the Trail Making Test;
for the adapted Trail Making Test determine a performance level for the user, the determination comprising utilization of the detected input from the user; and
for the adapted Trail Making Test when the determined performance level matches the required performance level present the remainder of the adapted Trail Making Test to the user.

8. System according to claim 7, wherein the processing unit is configured to utilize the detected input from the user as they trace the trail between targets as they undertake the subset of the Trail Making Test to determine a time for the user to complete the subset of the Trail Making Test, wherein the determined performance level comprises the time for the user to complete the subset of the Trail Making Test, and wherein the processing unit is configured to determine that the determined performance level matches the required performance level comprising a determination that the time for the user to complete the subset of the Trail Making Test is within a threshold time of an ideal time for the user to complete a subset of a Trail Making Test.

9. System according to claim 8, wherein the processing unit is configured to adapt the Trail Making Test to increase a task difficulty level of the Trail Making Test if the time for the user to complete the subset of the Trail Making Test is below the ideal time for the user to complete a subset of a Trail Making Test by more than the threshold time, and wherein the processing unit is configured to adapt the Trail Making Test to decrease a task difficulty level of the Trail Making Test if the time for the user to complete the subset of the Trail Making Test is above the ideal time for the user to complete a subset of a Trail Making Test by more than the threshold time.

10. System according to claim 9, wherein adaptation of the Trail Making Test comprises one or more of: a change in distance between two or more of the targets of the plurality of targets in the specific order when presented on the input/output unit; a change in a number of targets not in sequence that lie within a threshold distance of the trail linking the plurality of targets when presented on the input/output unit; a change in the symmetry of the plurality of targets when presented on the input/output unit; a change in intensity level and/or colour between adjacent targets when presented on the input/output unit.

11. System according to any of claims 8-10, wherein the processing unit is configured to determine from the input from the user as they trace a trail between targets as they undertake the subset of the Trail Making Test that the user has a deficiency with respect to a specific presentation area of the input/output unit less than the overall presentation area of the input/output unit, and wherein for the adapted Trail Making Test none of the subset of the plurality of targets or the remainder of the plurality of targets are presented on the input/output unit within the specific presentation area.

12. System according to any of claims 8-11, wherein the input/output unit is configured to detect brain activity of the user as they trace the trail between targets as they undertake the subset of the Trail Making Test, wherein determination of the performance level comprises utilization of the brain activity of the user as they trace the trail between targets as they undertake the subset of the Trail Making Test.

13. A Trail Making Test method (200), comprising:
presenting (210) by an input/output unit a calibration Trail Making Test to a user, wherein presentation of the calibration Trail Making Test comprises a plurality of targets being presented on the input/output unit;
undertaking (220) by the user the calibration Trail Making Test, where the user is requested to trace a trail linking the plurality of targets in a specific order, and wherein a task difficulty level increases progressively with respect to the trail linking the plurality of targets in the specific order;
detecting (230) by the input/output unit input from the user as they trace a trail between targets as they undertake at least some of the calibration Trail Making Test;
determining or selecting (240) by a processing unit an evaluation Trail Making Test for the user comprising utilizing the detected input from the user; and
presenting (250) by the input/output unit the evaluation Trail Making Test to the user, wherein presentation of the evaluation Trail Making Test comprises a plurality of targets being presented on the input/output unit, wherein to undertake the evaluation Trail Making Test the user is requested to trace a trail linking the plurality of targets in a specific order.

14. A Trail Making Test method (300), comprising:
presenting (310) by an input/output unit a subset of a Trail Making Test to a user, wherein presentation of the subset of the Trail Making Test comprises a subset of a plurality of targets being presented on the input/output unit;
undertaking (320) by the user the subset of the Trail Making Test, where the user is requested to trace a trail linking the subset of the plurality of targets in a specific order;
detecting (330) by the input/output unit input from the user as they trace a trail between targets as they undertake the subset of the Trail Making Test;
determining (340) by the processing unit a performance level for the user, the determining comprising utilization of the detected input from the user;
wherein the method comprises the processing unit:
if the determined performance level matches a required performance level presenting (350) the remainder of the Trail Making Test to the user, wherein presentation of the remainder of the Trail Making Test comprises a remainder of the plurality of targets being presented on the input/output unit, wherein to undertake the remainder of the Trail Making Test the user is requested to trace a trail linking the remainder of the plurality of targets in a specific order;
if the determined performance level does not match the required performance level carry out the following until the determined performance level matches the required performance level: adapting (360a-n) the Trail Making Test, wherein the adapting comprises utilizing the determined performance level;
for the adapted Trail Making Test presenting (310a-n) a subset of the Trail Making Test to the user, wherein presentation of the subset of the Trail Making Test comprises a subset of a plurality of targets being presented on the input/output unit,
for the adapted Trail Making Test undertaking (320a-n) by the user the subset of the Trail Making Test, where the user is requested to trace a trail linking the subset of the plurality of targets in a specific order;
for the adapted Trail Making Test detecting (330a-n) by the input/output unit input from the user as they trace a trail between targets as they undertake the subset of the Trail Making Test;
for the adapted Trail Making Test determining (340a-n) a performance level for the user, the determining comprising utilization of the detected input from the user; and
for the adapted Trail Making Test when the determined performance level matches the required performance level presenting (350) the remainder of the adapted Trail Making Test to the user.

15. A computer program element for controlling a system according to any of claims 1-6 which when executed by a processor is configured to carry out the method of claim 13; or for controlling a system according to any of claims 7-12 which when executed by a processor is configured to carry out the method of claim 12.
